**Europäisches Patentamt**

**European Patent Office**

**Office européen des brevets**

(19)

(11) Veröffentlichungsnummer: **0 068 160**
**B1**

# EUROPÄISCHE PATENTSCHRIFT

(12)

(45) Veröffentlichungstag der Patentschrift:
**29.10.86**

(21) Anmeldenummer: **82104748.7**

(22) Anmeldetag: **29.05.82**

(51) Int. Cl.⁴: **A 61 K 31/13** // C07C93/04

(54) Verwendung von 1-(p-Isopropoxyethoxymethyl-phenoxy)-3-isopropylamino-propan-2-ol zur Herstellung eines Arzneimittels zur Senkung des Augeninnendrucks.

(30) Priorität: **30.06.81 DE 3125636**

(43) Veröffentlichungstag der Anmeldung:
**05.01.83 Patentblatt 83/1**

(45) Bekanntmachung des Hinweises auf die Patenterteilung:
**29.10.86 Patentblatt 86/44**

(84) Benannte Vertragsstaaten:
**AT BE CH DE FR GB IT LI NL SE**

(56) Entgegenhaltungen:
**DE - A - 2 645 710**

(73) Patentinhaber: **Merck Patent Gesellschaft mit beschränkter Haftung, Frankfurter Strasse 250, D-6100 Darmstadt (DE)**

(72) Erfinder: **Harting, Jürgen, Dr., Rodinghweg 15, D-6100 Darmstadt (DE)**
Erfinder: **Fuchs, Andreas, Dr., Hebbelstrasse 36, D-6100 Darmstadt (DE)**

**Beschreibung**

Die Erfindung betrifft die Verwendung von 1-(p-2-Isopropoxyethoxymethyl-phenoxy)-3-isopropyl-amino-propan-2-ol (I) und/oder eines seiner physiologisch unbedenklichen Säureadditionssalze zur Herstellung eines Arzneimittels zur Senkung des Augeninnendrucks.

Zur Senkung des Augeninnendrucks und damit zur Glaukom-Behandlung werden insbesondere Pilocarpin und dessen Salze verwendet, die jedoch unerwünschte Nebenwirkungen wie Akkomodationsschwierigkeiten, Nachtblindheit und Beeinträchtigung des Reaktionsvermögens zeigen. Man hat daher versucht, andere Stoffe, insbesondere Betarezeptorenblocker wie Timolol (vgl. BE-PS 846 574) oder Bupranolol zur Glaukom-Behandlung einzusetzen. Auch diese sind jedoch nicht frei von Nachteilen. So treten pulmonale, kardiovaskuläre und zentralnervöse Nebenwirkungen auf, insbesondere auch Bronchokonstriktionen.

Der Erfindung lag die Aufgabe zugrunde, ein Arzneimittel zur Senkung des Augeninnendrucks und damit zur Glaukom-Behandlung, aufzufinden, das die Nachteile der bekannten Mittel nicht oder nur in geringerem Masse zeigt. Diese Aufgabe wurde dadurch gelöst, dass man die oben genannte Verbindung (I) zur Herstellung eines Arzneimittels zur Senkung des Augeninnendrucks verwendet.

Es wurde gefunden, dass die Verbindung I und ihre physiologisch unbedenklichen Salze den Augeninnendruck in hervorragender Weise senkt und ausserdem sehr gut verträglich ist. Nebenwirkungen, insbesondere Bronchokonstriktionen, treten gar nicht oder nur in sehr geringem Ausmass auf. Bei lokaler Anwendung am Auge von Kaninchen in Konzentrationen bis zu 40 mg/ml (wässrige Lösung, Fumarat) wurden keine Reizerscheinungen beobachtet.

Gegenstand der Erfindung ist die Verwendung der Verbindung I und ihrer physiologisch unbedenklichen Salze zur Herstellung eines Arzneimittels zur Senkung des Augeninnendrucks.

Die Substanz I und ihre physiologisch unbedenklichen Salze, insbesondere das bevorzugte Fumarat, sind aus der DE-OS 2 645 710 bekannt. Dort sind auch einige der pharmakologischen Wirkungen dieser Verbindungen beschrieben, insbesondere ihre isoprenalin-antagonistischen Wirkungen. Aus den dortigen Angaben ergab sich jedoch nicht in naheliegender Weise, dass diese Verbindungen bei gleichzeitiger sehr guter Verträglichkeit den Augeninnendruck hervorragend senken.

Die Verbindung I besitzt ein asymmetrisches C-Atom und kann daher in zwei optisch-aktiven Formen vorliegen. Beide Formen können erfindungsgemäss verwendet werden, ebenso ihre Gemische, insbesondere auch das optisch inaktive Racemat. Die S-(−)-Form ist bevorzugt.

Als physiologisch unbedenkliche Salze der Verbindung I kommen insbesondere Säureadditionssalze in Betracht. Diese leiten sich ab von anorganischen Säuren, z.B. von Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner von organischen Säuren, insbesondere aliphatischen, alicyclischen, araliphatischen, aromatischen oder heterocyclischen ein- oder mehrbasigen Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure.

Die Verbindung I und ihre physiologisch unbedenklichen Salze werden zur Senkung des Augeninnendrucks zweckmässig in Form üblicher pharmazeutischer Zubereitungen, wie sie für Ophthalmika gebräuchlich sind, verabreicht. Zu diesem Zweck kann I zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Diese Zubereitungen können als Ophthalmika in der Human- oder Veterinärmedizin verwendet werden. Als Trägerstoffe kommen organische oder anorganische Substanzen in Frage, die sich für die topische Applikation eignen und mit den Verbindungen nicht reagieren, beispielsweise Wasser, Gemische von Wasser und mit Wasser mischbaren Substanzen wie Alkanolen (z.B. Ethanol) und Aralkanolen (z.B. Benzylalkohol), pflanzliche Öle, Benzylalkohole, Polyalkylenglykole (z.B. Polyethylenglykole), Ester wie Glycerintriacetat, Ethyloleat oder Isopropylmyristat, Vaseline, Cellulosederivate wie Ethylcellulose oder Carboxymethylcellulose, Polyvinylpyrrolidon. Zur lokalen Anwendung dienen insbesondere Lösungen, vor allem in Form von Augentropfen, ferner Emulsionen, Suspensionen, Cremes oder Salben sowie auch Implantate. Diese Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, und/oder der Viskosität sowie gegebenenfalls Puffersubstanzen enthalten. Sie können, falls erwünscht, auch einen oder mehrere weitere Wirkstoffe enthalten, z.B. ein oder mehrere Vitamine oder andere den Augeninnendruck herabsetzende Stoffe wie Pilocarpin.

Die Verbindung I und ihre physiologisch unbedenklichen Salze werden in der Regel in Analogie zu bekannten, im Handel befindlichen Ophthalmika, insbesondere Glaukommitteln auf der Grundlage von Betarezeptorenblockern wie Timolol oder Bupranolol verabreicht, wie es beispielsweise in der BE-PS 846 574 beschrieben ist.

Vorzugsweise erfolgt die Applikation als Augentropfen in Form einer wässerigen Lösung, einer wässerigen (z.B. isotonischen) Kochsalzlösung oder einer öligen Lösung, deren Gehalt an I etwa zwischen 0,1 und 50, vorzugsweise zwischen 5 und 20 mg/ml beträgt und die einen pH-Wert zwischen etwa 5,5 und 8,5, insbesondere zwischen 6,0 und 7,5 besitzt. Falls I in Form des Fumarats in wässeriger Lösung (pH etwa 6,7) verwendet wird, ist der Zusatz von Puffer-Salzen nicht notwendig, aber auch nicht schädlich; geeignete Pufferzusätze sind beispielsweise Mononatrium-, Dinatrium-, Monokalium- und Dikaliumphosphat, Natriumborat, Borsäure, Natriumacetat, Mono-, Di- oder Trinatriumcitrat. Die Lösungen können ferner Konservierungsmittel bzw. Antiseptika enthalten, z.B. quartäre Ammoniumsalze wie Benzalkoniumchlorid, Phenylquecksilbersalze wie Phenylquecksilberacetat, Thiomersal, p-Hydroxybenzoesäure-methyl- oder propylester oder Benzylalkohol. Zur Erhöhung der Viskosität eignet sich z.B. der Zusatz von Cellulosederivaten, z.B. Methylcellulose. Als Einheitsdosis für das menschliche Auge werden zweckmässig etwa 0,001 bis 5 mg, vorzugsweise 0,005 bis 2 mg I appliziert.

In Salben oder Cremes beträgt die Konzentration an I vorzugsweise zwischen 0,05 und 1 Gewichtsprozent.

Als Implantate eignen sich beispielsweise Folien auf der Grundlage von physiologisch unbedenklichen hochmolekularen Stoffen, z.B. Cellulosederivaten wie Methyl-, Carboxymethyl-, Hydroxyethyl-, Hydroxypropyl-, Hydroxypropylmethylcellulose; Polyacrylaten wie Salzen der Polyacrylsäure, Polyethylacrylat, Polyacrylamid; Naturstoffen wie Gelatine, Alginate, Tragant; Polyvinylalkohol, Polyvinylpyrrolidon, Polyvinylmethylether; Polyethylenoxid. Die Folien können ausserdem Hilfsstoffe enthalten, z.B. Weichmacher, Konservierungsmittel oder Puffersubstanzen. Zur Verbesserung der Biegsamkeit können die Folien, insbesondere diejenigen auf der Grundlage von Cellulosederivaten, auch Wasser enthalten, in der Regel etwa 1 bis 40, vorzugsweise etwa 10 bis 20 Gewichtsprozent.

Die Folien können z.B. hergestellt werden, indem man den Wirkstoff zusammen mit dem Trägerstoff in einem inerten Lösungsmittel löst, die Lösung in eine geeignete Form mit grossem Flächeninhalt ausgiesst und das Lösungsmittel verdunsten lässt. Es ist auch möglich, ein festes Gemisch pulverförmiger Wirk- und Trägerstoffe in Abwesenheit eines Lösungsmittels zu schmelzen und die Schmelze zu einer Folie auszugiessen. Die Folien können z.B. Dicken zwischen etwa 0,25 und 15 mm, vorzugsweise zwischen 0,5 und 1,5 mm haben. Die erhaltene I-haltige Folie kann sodann in der gewünschten Weise zerschnitten werden, wobei die Implantate z.B. quadratische, rechteckige, kreis-, halbkreis- oder viertelkreisförmige oder ovale Formen haben können. Diejenigen Formen sind bevorzugt, die eine möglichst geringe Irritation des Auges bewirken.

Beispiel 1: Lösung (Augentropfen)
Man löst 1 g I-Fumarat und 0,01 g Benzalkoniumchlorid in sterilem destilliertem Wasser und füllt mit Wasser auf 100 ml auf. Die Lösung (pH 6,7) wird in Form von Augentropfen verwendet.

Beispiel 2: Salbe
Man vermischt 1 g I-Fumarat mit 200 g Vaseline.

Beispiel 3: Lösung (Augentropfen)
Man löst 0,5 g I-Hydrochlorid, 1,2 g Natriumchlorid und 0,002 g Phenylquecksilberacetat in Wasser, stellt mit Natronlauge auf pH 8,2 ein und füllt mit Wasser auf 100 ml auf.

Beispiel 4: Lösung (Augentropfen)
Man löst 2 g I-Methansulfonat, 1 g Dinatriumhydrogenphosphat-12-hydrat, 0,002 g Phenylquecksilberacetat und 0,4 g Methylcellulose in Wasser, stellt auf pH 7,6 ein und füllt mit Wasser auf 100 ml auf.

Beispiel 5: Lösung (Augentropfen)
Man löst 1,5 g I(Base) und 1 g Benzylalkohol in Erdnussöl und füllt mit Erdnussöl auf 100 ml auf.

Beispiel 6: Implantat
Man giesst eine Lösung von 1 g I-Fumarat und 12 g Hydroxypropylcellulose in 60 ml Methanol auf eine Teflonplatte, so dass nach dem Verdampfen des Lösungsmittels bei 20° eine dünne Folie entsteht. Anschliessend schneidet man in kleine Stücke der gewünschten Grösse, die 3 Stunden in einer Feuchtigkeitskammer (88% Feuchtigkeit bei 30°) aufbewahrt und danach einzeln steril eingesiegelt werden. Jedes Stück enthält 5 mg I-Fumarat.

**Patentanspruch**

Verwendung von 1-(p-2-Isopropoxyethoxymethyl-phenoxy)-3-isopropylamino-pronan-2-ol und/oder einem seiner physiologisch unbedenklichen Salze zur Herstellung eines Arzneimittels zur Senkung des Augeninnendrucks.

**Claim**

Use of 1-(p-2-isopropoxyethoxymethyl-phenoxy)-3-isopropylamino-propane-2-ol and/or one of its physiologically unobjectionable salts for the manufacture of a medicament for decreasing the intra-ocular pressure.

**Revendication**

Utilisation du 1-(p-2-isopropoxyéthoxyméthyl-phénoxy)-3-isopropylamino-propane-2-ol et/ou de ses sels acceptables pour l'usage pharmaceutique pour la préparation d'un médicament servant à diminuer la pression interne de l'œil.